(19) Europäisches Patentamt
European Patent Office
Office européen des brevets

(11) Publication number: **0 127 090 A2**

# (12) EUROPEAN PATENT APPLICATION

(21) Application number: 84105691.4

(22) Date of filing: 18.05.84

(51) Int. Cl.³: C 07 C 103/52 //A61K37/02

(30) Priority: 20.05.83 US 496756

(43) Date of publication of application: 05.12.84 Bulletin 84/49

(84) Designated Contracting States: AT BE CH DE FR GB IT LI LU NL SE

(71) Applicant: USV PHARMACEUTICAL CORPORATION
1 Scarsdale Road
Tuckahoe New York(US)

(72) Inventor: Huang, Fu-Chih
611 Knoll Road
Boonton New Jersey(US)

(72) Inventor: Suh, John T.
59 Stanwich Road
Greenwich Connecticut(US)

(72) Inventor: Skiles, Jerry W.
1 Consulate Drive
Tuckahoe New York(US)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22(DE)

## (54) Angiotensin-converting enzyme inhibitors.

(57) Compounds of the formula

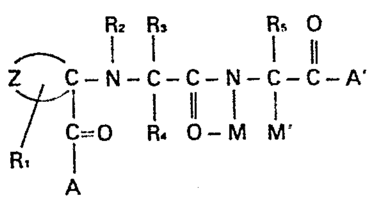

and their pharmaceutically acceptable salts, wherein the substituents are as defined herein, having antihypertensive activity.

EP 0 127 090 A2

Croydon Printing Company Ltd

PATENTANWÄLTE
0127090
MAXIMILIANSTR. ... MÜNCHEN 22

# ANGIOTENSIN-CONVERTING ENZYME INHIBITORS

This application relates to compounds, their pharmaceutically acceptable salts, and pharmaceutical preparations made therefrom, having biological activity as inhibitors of the enzymatic conversion of angiotensin I to angiotensin II. As such, the products comprising the present invention have utility in the treatment of hypertension in subjects suffering therefrom.

Broadly stated, the present invention relates to compounds of the formula:

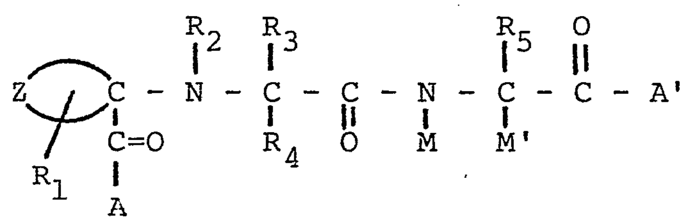

(1)

and their pharmaceutically acceptable salts, wherein

A and A' are independently hydroxy, alkoxy, alkenoxy, mono- or dialkylaminoalkoxy, acylaminoalkoxy, acyloxyalkoxy, aryloxy, arylalkyloxy, amino, alkylamino, dialkylamino, arylalkylamino, hydroxyamino, or substituted aryloxy, or substituted arylalkoxy wherein the substituent is methyl, halo or methoxy;

$R_1$ is hydrogen, alkyl, aryl, aralkyl, fused cycloalkyl-aryl, or fused aryl-cycloalkyl;

$R_2$, $R_3$, $R_4$ and $R_5$ are independently hydrogen, alkyl, alkenyl, alkynyl, aryl, fused cycloalkyl-aryl, fused aryl-cycloalkyl, aralkyl, cycloalkyl, or heterocyclic, or

$R_2$ and $R_3$ when taken together with the nitrogen and carbon atoms to which they are respectively attached form an N-heterocyclic ring containing from 3 to 5 carbon atoms or 2 to 4 carbon atoms and a sulfur atom;

M is hydrogen, alkyl, alkenyl, alkynyl, cycloalkyl, acyl, cycloalkyl-alkyl, polycycloalkyl, polycycloalkyl-alkyl, aryl, aralkyl, heteroaryl, heteroaryl-alkyl, hetero-cycloalkyl, heterocycloalkyl-alkyl, fused polycyclic aryl, fused cycloalkyl-aryl, fused aryl-cycloalkyl, fused aryl-cycloalkyl-alkyl, fused heteroaryl-cycloalkyl, fused heteroaryl-cycloalkyl-alkyl, alkoxyalkyl, alkylthioalkyl, alkylaminoalkyl, or dialkylaminoalkyl,

M' is hydrogen, loweralkyl, cycloalkyl, phenyl, phenylalkyl, hydroxyphenylalkyl, hydroxyalkyl, aminoalkyl, guanidinoalkyl, imidazolylalkyl, indolylalkyl, mercaptoalkyl or alkylthioalkyl, or

M and M' when connected together form a saturated or unsaturated bridge of from 2 to 5 carbon atoms; from 2 to 4 carbon atoms and one oxygen or sulfur atom; fused cycloalkylalkylene; or substituted or unsubstituted fused aralkylene in which the substituent is hydroxy, alkoxy, or alkyl; and

Z contains 2, 3, 4 or 5 carbon atoms which optionally may be interrupted by an O, N, or S atom, and is saturated or contains at most one double bond between adjacent carbon atoms;

wherein the alkyl, alkenyl, and alkynyl groups may include the substituents hydroxy, alkoxy, amino, mono- or dialkylamino, thio, or alkylmercapto, the cycloalkyl groups may include the substituents alkyl, hydroxy, alkylamino, nitro or trifluoromethyl; and the aryl, fused ring, and heterocyclic groups may include the substituents carboxyl, alkoxycarbonyl, alkyl, hydroxy, alkoxy, hydroxyalkyl, halo, haloalkyl, mercapto, alkylthio, mercaptoalkyl, amino, alkylamino, aminoalkyl, nitro, methylenedioxy, or trifluoromethyl.

Preferred compounds of the present invention are those of the general formula given above in which $R_1$, $R_2$, $R_3$, $R_4$ and $R_5$ are each hydrogen or lower alkyl; M is phenethyl, 5-indanyl, 2-indanyl, or tolyl, or is taken together with M' and the N and C atoms to which M and M' are respectively atttached, to form an alkylene bridge 2 to 4 carbon atoms in length, or a dihydroindole or tetrahydroisoquinoline ring structure; Z forms a cyclopropyl, cyclobutyl, cyclopentyl or cyclohexyl ring; and A and A' are each hydroxy or lower alkoxy.

The alkyl groups per se and the alkyl moieties in alkoxy, aralkyl, cycloalkyl, aminoalkyl, acyl, and the like, may be straight-chained or branched and preferably contain from 1 to 20 carbon atoms. Such groups include methyl, ethyl, propyl, isopropyl, butyl, isobutyl, tertiary-butyl, amyl, iso-amyl, hexyl, octyl, dododecyl, and the like. Preferably, the alkyl groups are lower alkyl containing from 1 to 6 carbon atoms.

The alkenyl and alkynyl groups and moieties can also be straight or branched-chained groups containing from 2 to 20, and preferably 2 to 6, carbon atoms. Such groups include vinyl, ethynyl, propenyl, alkyl, isopropenyl, and the like.

The acyl group includes groups such as alkanoyl, aroyl, sulfonyl, carbamoyl, and the like.

The alkyl, alkenyl, and alkynyl groups can include substituents such as hydroxy, alkoxy, amino,

alkylamino, dialkylamino, mercapto, alkylmercapto, halo, and the like.

The cycloalkyl, polycycloalkyl, aryl, heteroaryl, aralkyl, fused aryl-cyclalkyl groups and the like contain from 3 to 16 carbon atoms and may include substitutents such as lower alkyl, alkenyl, alkynyl, hydroxy, mercapto, amino, keto-oxygen, alkoxy, alkylmercapto, alkylamino, dialkylamino, halo, trifluoromethyl and the like. The groups include such radicals as cyclopropyl, cyclobutyl, cyclopentyl, cyclohexyl, cycloheptyl, adamantyl, norbornyl, phenyl, tolyl, benzyl, phenethyl, dimethoxyphenyl, hydroxybenzyl, indanyl, naphthyl, tetrahydronaphthyl, decanhydronaphthyl, pyridyl, quinolyl, pyrrolidyl, pyrrolyl, morpholinyl, furyl, furfuryl, tetrahydrofurfuryl, benzimidazolyl, thienyl, imidazolyl, and the like.

The halo groups include fluoro, chloro, bromo and iodo.

The particularly preferred compounds of the invention are those in which at least one sulfamyl group is present; these compounds also possess significant diuretic activity in addition to ACE inhibition. Of particular value are those compounds which include sulfamyl-substituted phenyl groups and benzothiadiazine-1, 1-dioxides and the 3,4-dihydro derivatives thereof.

It will be understood by those skilled in the art that compounds within the present invention can have one or more asymmetric carbons each of which is in the (R) or (S) configuration. All combinations of configurations are within the scope of the invention; the preferred

compounds are those in the (S), (S,S), or (S,S,S) form.

The products of the present invention can be prepared by amide forming reaction of an acid of formula A or amide forming derivative thereof

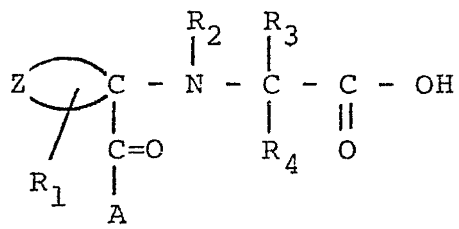
(A)

with a compound of formula B

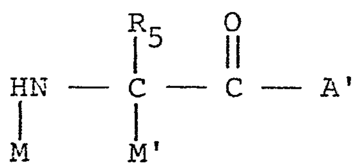
(B)

Alternatively, the present compounds can be formed by condensation of a compound of formula C

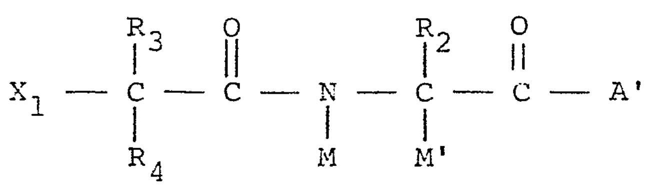
(C)

in which $X_1$ is halogen or amino, with a compound of formula D

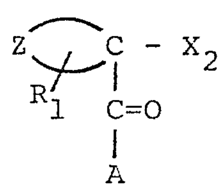
(D)

in which $X_2$ is halogen or amino, under conditions of hydrogen halide removal. Obviously, when $X_1$ is halogen, $X_2$ must be amino and $X_1$ is amino, $X_2$ must be halogen for hydrogen halide elimination and compound formation.

In the aforementioned reaction sequences, it is preferred to employ blocking groups to preclude unwanted reactions at either amino or carboxyl functions. The blocking groups such as carbobenzyloxy and 2,2,2-trichloroethoxycarbonyl for amino groups and ester groups such as ethoxy, t-butoxy and benzyloxy for carboxyl groups are usually removed after product formation, as desired.

The amide forming conditions referred to herein involve the use of known derivatives of the described acids, such as the acyl halides, anhydrides, mixed anhydrides, lower alkyl esters, carbodiimides, carbonyl diimidazoles, and the like. The reactions are carried out in organic solvents such as acetonitrile, tetrahydrofuran, dioxane, acetic acid, methylene chloride, ethylene chloride and similar such solvents. The amide forming reaction will occur at room temperature or at elevated temperature. The use of elevated temperature is for convenience in that it permits somewhat shortened reaction periods. Temperatures ranging from 0°C. up to reflux temperature of the reaction system can be used. As a further convenience the amide forming reaction can be effected in the presence of a base such as tertiary organic amines, e.g., trimethylamine, pyridine, picolines and the like, particularly where hydrogen halide is formed by the amide-forming reaction,

e.g., acyl halide and amino compound. Of course, in those reactions where hydrogen halide is produced, any of the commonly used hydrogen halide acceptors can also be used.

In the condensation of an alpha haloacid derivative, similar reaction conditions, solvents and hydrogen halide acceptors can be used as for amide formation.

Various substituents on the present new compounds can be present in the starting compounds or added after formation of the amide products by the known methods of substitution or conversion reactions. Thus, the nitro group can be added to the final product by nitration of the aromatic ring and the nitro group converted to other groups, such as amino by reduction, and halo by diazotization of the amino group and replacement of the diazo group. Other reactions can be effected on the formed amide product. Amino groups can be alkylated to form mono and dialkylamino groups, mercapto and hydroxy groups can be akylated to form corresponding ethers. Thus, substitution or alteration reactions can be employed to provide a variety of substituents throughout the molecule of the final products. Of course, reactive groups where present should be protected by suitable blocking groups during any of the aforesaid reactions particularly the condensation reactions to form the amide linkages.

The products are obtained typically as a mixture of diastereoisomers which can be separated by standard methods of fractional crystallization or chromatography.

The compounds of this invention form acid salts with various inorganic and organic acids which are also within the scope of the invention. The pharmaceutically acceptable acid addition salts of the compounds of the present invention may be prepared by conventional reactions by reacting the free amino acid or amino ester with an appropriate acid providing the desired anion, either in a solvent or medium in which the salt is insoluble, or in water and removing the water by freeze drying. The salts of strong acids are preferred. As exemplary, but not limiting, of pharmaceutically acceptable acid salts are the salts of hydrochloric, hydrobromic, sulfuric, nitric, acetic, furmaric, malic, maleic and citric acids.

The compounds of this invention also form salts with bases. Suitable such salts include salts with alkali and alkaline earth metals such as sodium, potassium, magnesium and calcium as well as iron, and salts with ammonia and amines, and quaternary ammonium salts.

The action of the enzyme renin or angiotensinogen, a pseudoglobulin in blood plasma, produces the decapeptide angiotensin I. Angiotensin I is converted by angiotensin converting enzyme (ACE) to the octapeptide angiotensin II. The latter is an active pressor substance which has been implicated as the causative agent in various forms of hypertension in various mammalian species, e.g., rats and dogs. The compounds of this invention intervene in the renin-to-angiotensin I-to-angiotensin II sequence by inhibiting angiotensin I

converting enzyme and reducing or eliminating the formation of the pressor substance angiotensin II and therefore, are useful in reducing or relieving hypertension. Thus, by the administration of a composition containing one or a combination of compounds of formula (1) or pharmaceutically acceptable salts thereof, hypertension in the species of mammal suffering therefrom is alleviated. A single dose, or preferably two to four divided daily doses, provided on a basis of about 0.1 to 100 mg per kilo-gram per day, preferably about 1 to 50 mg per kilogram per day, is appropriate to reduce blood pressure. The substance is preferably administered orally, but a parenteral route such as subcutaneously, intramuscularly, intravenously or intraperitonealy can also be employed.

The compounds of the invention can be utilized to achieve the reduction of blood pressure by formulating one or more of them in compositions such as tablets, capsules or elixirs for oral administration or in sterile solutions or suspensions for perenteral administration. About 10 to 500 mg of a compound or mixture of compounds of formula (1) or physiologically acceptable salt thereof is compounded with a physiologically acceptable vehicle, carrier, excipient, binder, preservative, stabilizer, flavor, etc., in a unit dosage form as called for by accepted pharmaceutical practice. The amount of active substance in these compositions or preparations is such that a suitable dosage in the range indicated is obtained.

Illustrative of the adjuvants which may be incorporated in tablets, capsules and the like are the following: a binder such as gum tragacanth, acacia, corn starch or gelatin; an excipient such as dicalcium phosphate; a disintegrating agent such as corn starch, potato starch, alginic acid and the like; a lubricant such as magnesium stearate; a sweetening agent such as sucrose, lactose or saccharin; a flavoring agent such as peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets may be coated with shellac, sugar or both. A syrup or elixir may contain the active compound, sucrose as a sweetening agent, methyl and propyl parabens as preservatives, a dye and a flavoring such as cherry or orange flavor.

Sterile compositions for injection can be formulated according to conventional pharmaceutical practice by dissolving or suspending the active substance in a vehicle such as water for injection, a naturally occurring vegetable oil like sesame oil, coconut oil, peanut oil, cottonseed oil, etc., or a synthetic fatty vehicle like ethyl oleate, and the like. Buffers, preservatives, antioxidants and the like can be incorporated as required.

Specific embodiments of the invention are illustrated in the following Examples.

0127090

EXAMPLE I

N-(1-carbethoxy-1-cyclopentyl)-alanyl-proline t-butyl ester

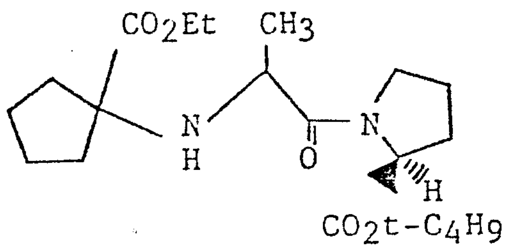

(I)

A mixture of ethyl 1-amino-1-cyclopentanoate (10 g), benzyl 2-iodopropionate (20 g), and triethyl amine (6.43 g) in 70 ml of dimethyl formamide was stirred overnight at room temperature and heated at 60° for 1 hour. The solvent was removed under vacuum. The residue was acidified to pH 2 and extracted with ether. The aqueous solution was neutralized to pH 10 and then extracted with ethyl acetate. The combined ethyl acetate extracts were dried and evaporated to dryness. Purification by dry column chromatography gave 9 g of the intermediate product (I-A), ethyl 1-N-(benzyl 2-propionate)-1-amino-1-cyclopentane carboxylate:

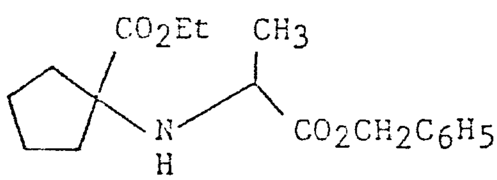

(I-A)

Ten grams of the product (I-A) was hydrogenated with 1.2 g of 5% Pd/C in 150 ml of ethanol at 40 psi for 2.5 hr. After filtration the solvent was evaporated to give 5.5 g of intermediate product (I-B), ethyl 1-N-(2-propionic acid)-1-amino-1-cyclopentane carboxylate:

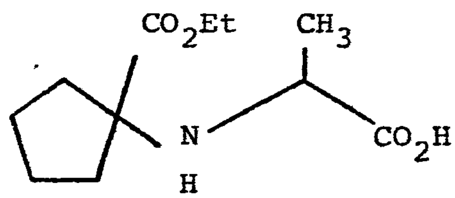

(I-B)

The product (I-B) (3.3 g) in 50 ml of $CH_2Cl_2$ was treated with carbonyl di-imidazole (2.34 g) at room temperature. The reaction mixture was stirred for 30 minutes and then L-proline t-butyl ester (2.5 g) in 10 ml of $CH_2Cl_2$ was added dropwise. After stirring overnight at room temperature, the organic solution was washed well with water, dried and evaporated to give 4.5 g of crude product. Purification by dry column chromatography gave 2.2 g of oily product.

EXAMPLE II

N-(1-carboxy-1-cyclopentyl)-alanyl-proline

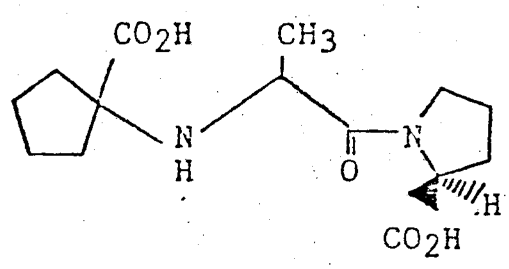

(II)

The peptide I from Example I (2.2 g) in 50 ml of ether was treated with dry HCl gas for 2 hours at 0°C. The solvent was evaporated and the residue was treated with ethanolic KOH (10 ml, 1 N) in 15 ml of dioxane. After evaporation of solvent, the residue was purified on Dowex 50 X 2 - 100 (80 ml volume). The peptide was eluted with 3% pyridine. The combined ninhydrin positive fraction was lyophilized to give a white solid product, mp 142-5°.

EXAMPLE III

N-(1-carbethoxy-1-cyclohexyl)-alanyl-proline t-butyl ester

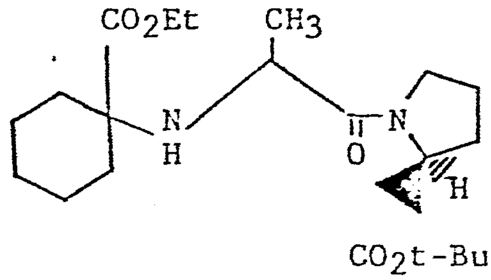

(III)

A mixture of ethyl 1-amino-1-cyclohexylcarboxylate (10 g) benzyl 2-iodopropionate (18.5 g), and triethyl amine (6.46 g) in 50 ml of DMF was stirred at 50°C. overnight. DMF was removed under reduced pressure. The residue was dissolved in 1 N HCl solution, then washed with ether. The aqueous layer was basified with NaOH to pH 10 and was then extracted with methylene chloride. The combined methylene chloride extracts were dried with magnesium sulfate and concentrated. Purification with dry column chromatography gave 8 g of the intermediate product (III-A), ethyl 1-N-(benzyl-2-propionate)-1-amino-1-cyclohexane carboxylate

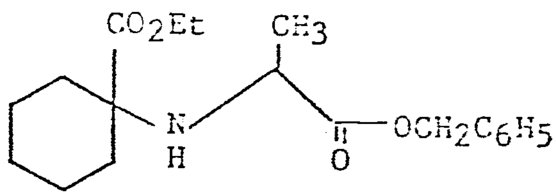

(III-A)

A solution of 4.6 g of compound (III-A) and 1 g of 10% Pd/C in 100 ml of ethanol was hydrogenated at 50 psi for 5 hours. After filtration and evaporation of solvent, 3 g of product (III-B) was obtained, ethyl 1-N-(2-propionic acid)-1-amino-1-cyclohexane carboxylate:

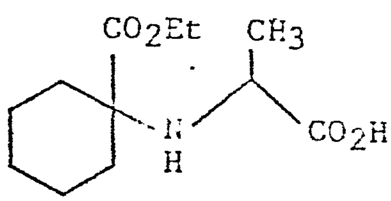

(III-B)

To a solution of 2.4 g of a compound (III-B) in 50 ml of dry tetrahydrofuran was added 1.62 g of carbonyldiimidazole. The solution was stirred at room temperature for 40 minutes. L-proline t-butyl ester (1.71 g) was then added and stirring was continued overnight. After evaporation of solvent, the residue was extracted with methylene chloride. The combined organic extracts were dried with magnesium sulfate and concentrated. Purification by dry column chromatography gave 1.8 g of the desired product (III).

0127090

EXAMPLE IV

N-(1-carbethoxy-1-cyclohexyl)-alanyl-proline

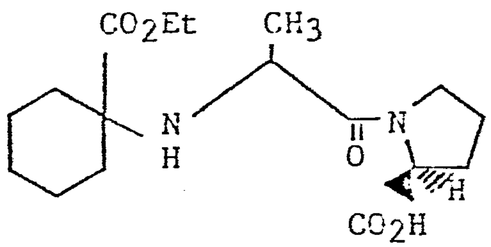

.HCl (IV)

An etheral solution of compound (III) (1.8 g) in 100 ml of ether was treated with dry HCl gas at 0°C. for two hours. Solvent was evaporated. Repeated reprecipitation with methyelen chloride and ether gave 1.5 g of hygroscopic product (IV).

0127090

## EXAMPLE V

N-(1-carboxy-1-cyclohexyl)-alanyl-proline

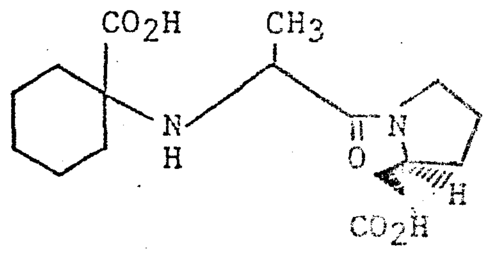

(V)

Compound (IV) from Example IV (0.5 g) in 10 ml of 1 NaOH solution was stirred at room temperature for three days. The reaction mixture was neutralized to pH 7 and lyophilized. Purification through Dowex 50 X 2 - 100 resin to remove salts and neutral products gave 0.2 g of product (V).

EXAMPLE VI

N-(1-carbethoxy-1-cyclohexyl)-alanyl-(1,2,3,4-tetrahydro-3-carbomethoxy)-isoquinoline

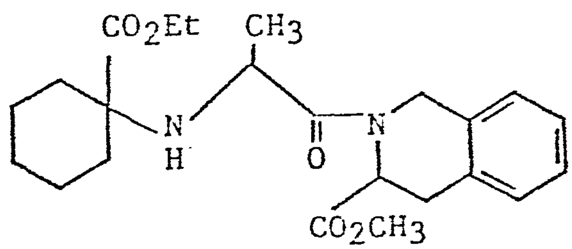

(VI)

To a solution of 1.5 g of compound (III-B) from Example III in 20 ml of methylene chloride was added 10 g of carbonyldiimidazole. The solution was stirred at room temperature for 40 minutes, and then 1.41 g of methyl tetrahydroisoquinoline-3-carboxylate was was added. The reaction was continued overnight. The organic solution was washed with water, then with sodium bicarbonate solution, dried with magnesium sulfate, and concentrated. Purification by dry column chromatography gave 1.4 g of product (VI).

0127090

## EXAMPLE VII

N-(1-carboxy-1-cyclohexyl)-alanyl-(1,2,3,4-tetrahydro-3-carboxyl)-isoquinoline

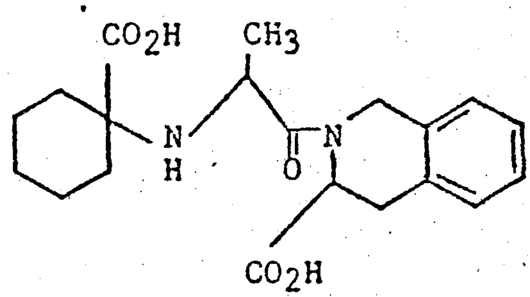

(VII)

Compound (VI) from Example VI (1.4 g) in 10 ml of 1 N NaOH solution was stirred at room temperature for 3 days. The reaction mixture was neutralized to pH 7 and then lyophilized. Purification through Dowex 50 X 2 - 100 resin (100 ml) to remove salts and neutral products gave 0.6 g of product (VII).

0127090

EXAMPLES VIII - X

In a manner wholly analogous to that employed above, the following compounds are also prepared.

VIII: N-(1-carbethoxy-1-cyclopropyl)-alanyl-proline t-butyl ester.

IX: N-(1-carbethoxy-1-cyclopropyl)-alanyl-proline hydrochloride.

X: N-(1-carbethoxy-1-cyclopentyl)-lysinyl proline.

WHAT IS CLAIMED IS:

1. Compounds of the formula:

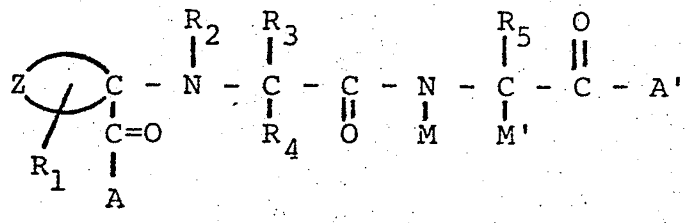 (1)

and their pharmaceutically acceptable salts, wherein

A and A' are independently hydroxy, alkoxy, alkenoxy, mono- or dialkylaminoalkoxy, acylaminoalkoxy, acyloxyalkoxy, aryloxy, arylalkyloxy, amino, alkylamino, dialkylamino, arylalkylamino, hydroxyamino, or substituted aryloxy, or substituted arylalkoxy wherein the substituent is methyl, halo or methoxy;

$R_1$ is hydrogen, alkyl, aryl, aralkyl, fused cycloalkyl-aryl, or fused aryl-cycloalkyl;

$R_2$, $R_3$, $R_4$ and $R_5$ are independently hydrogen, alkyl, alkenyl, alkynyl, aryl, fused cycloalkyl-aryl, fused aryl-cycloalkyl, aralkyl, cycloalkyl, or heterocyclic, or

$R_2$ and $R_3$ when taken together with the nitrogen and carbon atoms to which they are respectively attached form an N-heterocyclic ring containing from 3 to 5 carbon atoms or 2 to 4 carbon atoms and a sulfur atom;

0127090

M is hydrogen, alkyl, alkenyl, alkynyl, cycloalkyl, acyl, cycloalkyl-alkyl, polycycloalkyl, polycycloalkyl-alkyl, aryl, aralkyl, heteroaryl, heteroaryl-alkyl, hetero-cycloalkyl, heterocycloalkyl-alkyl, fused polycyclic aryl, fused cycloalkyl-aryl, fused aryl-cycloalkyl, fused aryl-cycloalkyl-alkyl, fused heteroaryl-cycloalkyl, fused heteroaryl-cycloalkyl-alkyl, alkoxyalkyl, alkylthioalkyl, alkylaminoalkyl, or dialkylaminoalkyl,

M' is hydrogen, loweralkyl, cycloalkyl, phenyl, phenylalkyl, hydroxyphenylalkyl, hydroxyalkyl, aminoalkyl, guanidinoalkyl, imidazolylalkyl, indolylalkyl, mercaptoalkyl or alkylthioalkyl, or

M and M' when connected together form a saturated or unsaturated bridge of from 2 to 5 carbon atoms; from 2 to 4 carbon atoms and one oxygen or sulfur atom; fused cycloalkylalkylene; or substituted or unsubstituted fused aralkylene in which the substituent is hydroxy, alkoxy, or alkyl; and

Z contains 2, 3, 4 or 5 carbon atoms which optionally may be interrupted by an O, N, or S atom, and is saturated or contains at most one double bond between adjacent carbon atoms;

wherein the alkyl, alkenyl, and alkynyl groups may include the substituents hydroxy, alkoxy, amino, mono- or dialkylamino, thio, or alkylmercapto, the cycloalkyl groups may include the substituents alkyl, hydroxy, alkylamino, nitro or trifluoromethyl; and the aryl, fused ring, and heterocyclic groups may include the substituents carboxyl, alkoxycarbonyl, alkyl, hydroxy, alkoxy, hydroxyalkyl, halo, haloalkyl, mercapto, alkylthio, mercaptoalkyl, amino, alkylamino, aminoalkyl, nitro, methylenedioxy, or trifluoromethyl.

2. The compounds and salts of Claim 1 wherein M and M' are connected together to form together with the N and C to which they are connected a tetrahydro-isoquinoline ring, a dihydro-indole ring, or a proline ring.

3. The compounds and salts as in Claim 1 or Claim 2 wherein M is phenethyl, 5-indanyl, 2-indanyl, or tolyl.

4. The compounds and salts as in any of Claims 1-3 wherein Z is $-(CH_2)_2-$, $-(CH_2)_3-$, $-(CH_2)_4-$ or $-(CH_2)_5-$.

5. The compounds and salts as in any of Claims 1-4 wherein at least one of $R_2$, $R_3$, and $R_5$ is hydrogen and $R_4$ is alkyl or aminoalkyl.

6. Compounds and salts according to Claim 1 of the formula

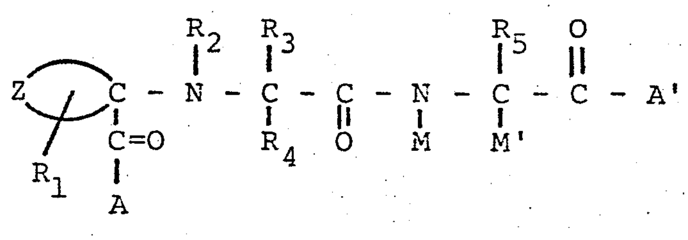

wherein

$R_4$ is alkyl or aminoalkyl,

A and A' are independently hydroxy, alkoxy, or benzyloxy,

M and M' are connected together to form an alkylene bridge or from 2 to 4 carbon atoms or together with the N and C to which they are connected form a 1,2,3,4-tetrahydroisoquinoline ring, and

Z is $-(CH_2)_2-$, $-(CH_2)_3-$, $-(CH_2)_4-$ or $-(CH_2)_5-$.

7. A product as in Claim 1 which is one of the following:

N-(1-carboxy-1-cyclohexyl)-alanyl-(1,2,3,4-tetrahydro-3-carboxy)-isoquinoline;

N-(1-carbethoxy-1-cyclohexyl)-alanyl-(1,2,3,4-tetrahydro-3-carboxy-isoquinoline;

N-(1-carbethoxy-1-cyclohexyl)-alanyl(1,2,3,4-tetrahydro-3-carbomethoxy)-isoquinoline;

N-(1-carboxy-1-cyclopentyl)-alanyl-L-proline;

N-(1-carbethoxy-1-cyclopentyl)-alanyl-L-proline;

N-(1-carbethoxy-1-cyclopentyl)-alanyl-L-proline-t-butyl ester;

N-(1-carbethoxy-1-cyclohexyl)-alanyl-L-proline;

N-(1-carbethoxy-1-cyclohexyl)-alanyl-L-proline-t-butyl ester;

N-(1-carboxy-1-cyclohexyl)-alanyl-L-proline;

N-(1-carbethoxy-1-cyclopropyl)-alanyl proline t-butyl ester;

N-(1-carbethoxy-1-cyclopropyl)-alanyl proline;

N-(1-carbethoxy-1-cyclopentyl)-lysinyl proline;

and pharmaceutically acceptable salts thereof.